# EUROPEAN PATENT APPLICATION

(11) **EP 1 125 933 A1**
(43) Date of publication of application: **22.08.2001**
(21) Application number: 00102560.0
(22) Date of filing: 07.02.2000
(51) Int. Cl.: C07D 301/10, B01J 23/52, B01J 23/66

(54) **Epoxidation catalyst containing metals of the lanthanoide series**

(71) Applicant: Bayer Aktiengesellschaft, 51368 Leverkusen (DE)
(72) Inventor: Cunningham, A. H. Derek, Dr., 51061 Köln (DE); Zumaqué, Harry, Dr., 53117 Bonn (DE)

(57) **Abstract**

The invention is directed towards a process for the epoxidation of olefins, using molecular oxygen and hydrogen, characterized in that, as catalyst, a compound comprising gold, preferably in nanometer size, on a support material, in which the support material contain one or more element(s) from the lanthanoide series is applied, and a compound comprising gold, preferably in nanometer size, on a support material, in which the support material contain one or more element(s) selected from the group consisting of the metals having the atomic number 58-71 of which Cerium and Neodymium are excluded, a process for the preparation of said compounds and a method of catalyzing a chemical reaction comprising conducting said chemical reaction in the presence of said compound.

## Description

### Background of the invention

Direct gas phase partial oxidation of olefins by molecular oxygen to epoxides is long considered one of the most important reactions in commercial catalysis. Because of the importance of epoxides in the polyurethane industry, many attempts have been made to make epoxides by various means, some of which are commercialized. To produce epoxides from olefins containing more than two carbon atoms most production techniques use hydrogen peroxide or chlorohydrin as an oxidant. European patent (EP-A1-0 930 308) for example describes the use of ion exchanged titanium silicate for the production of epoxides in the presence of hydrogen peroxide, or chlorohydrin as the oxidant. More recently, US-A-5,623,090 describes a new class of materials that may allow the direct production of epoxides such as propylene oxide directly from the olefin propylene using molecular oxygen, while in the co-presence of hydrogen. In this patent it is claimed that when gold is deposited on titanium, specifically anatase Titanium dioxide the direct gas phase partial oxidation of propylene to propylene oxide takes place.

Though the Au/titanium oxide system is still far from commercialization, and exhibits poor reaction yields, what separates gold from previous known inventions is the higher selectivities observed for the epoxidation of olefins with 3 or greater carbons, an example of such being propylene. Silver based catalyst systems, for example, despite showing good yields and selectivities for ethylene oxide production, fail to give high or promising activities for propylene conversion. Subsequent patents since the work of Hayashi and Haruta (see Hayashi et al., Symposium on heterogeneous Hydrocarbon Oxidation, presented at the Div. Of Petroleum Chemistry, 211^{th} National Meeting, American Chem. Soc., New Orleans, LA, March 24-29 1996) have therefore mainly concentrated on the use of gold in conjunction with Titanium WO 97/34692-A1, WO 98/00413-A1, WO 98/00414-A1. The exception is patent EP-A1-0 940 393, that employs gold in the co-presence of the element Zr. Thus, the current understanding of the art is that the number of active species which can aid the partial oxidation of olefinic material is limited. Furthermore Mohr, Hofmeister, Lucas and Claus disclose in Chem.-Ing.-Techn. 71, p. 869-873 (1999) the use of Au/CeO₂, Au/Y₂O₃, Au/Nd₂O₃ hydration catalysts and Rodemerck, Ignaszewski, Lucas and Claus disclose in Chem.-Ing.-Techn. 71, p. 873-877 (1999) the use of Au/CeO₂, Au/Y₂O₃, Au/Nd₂O₃ for the oxidation of CO. Both documents are silent about the favorable use of catalysts containing metals of the lanthanoide series and gold as epoxidation catalysts.

### Summary of the invention

The inventions described herein involve a process for the epoxidation of olefins, using molecular oxygen and hydrogen, characterized in that, as catalyst, a compound comprising gold, preferably in nanometer size, on a support material, in which the support material contain one or more element(s) from the lanthanoide series is applied. All catalysts operate free of the element Titanium. These finding are surprising, in light of the fact that in the last three years of intensive research very few other catalysts systems containing gold have been discovered for the epoxidation reaction of olefins. The invention shows in several cases good stability of the catalysts over extended time periods.

Another object of the invention are compounds comprising gold, preferably in nanometer size, on a support material, in which the support material contain one or more element(s) selected from the group consisting of the metals having the atomic number 58-71 of which, in this object, Cerium and Neodymium are excluded.

Yet another object of the invention is a method of catalyzing a chemical reaction through conducting said chemical reaction in the presence of a compound comprising gold, preferably in nanometer size, on a support material, in which the support material contain one or more element(s) selected from the group consisting of the metals having the atomic number 58-71 of which Cerium and Neodymium are excluded.

Yet another object of the invention is a process for the preparation of the invented compounds, characterized in that, gold particles of nanometer size are deposited on a support material in which the support material contain one or more element(s) from the lanthanoide series.

Yet another object of the invention is a process for the preparation of the invented compounds, characterized in that, compounds comprising gold particles of nanometer size on a support material in which the support material contain one or more element(s) from the lanthanoide series are prepared via a sol-gel-process.

### Detailed description of the invention

As with many catalysts currently used in partial oxidation reactions, although any olefin can be used, the catalysts described within are apparently best able to activate the epoxidation of light olefins between C3 and C6, especially propene and butene. In the olefin the number of carbon-carbon double bonds contained is normally one but systems containing more than one can also be used. Examples to which the invention may be applied to include, ethylene, propylene, 1-butene, 2-butene, isobutylene, 1-pentene, 2-pentene, butadiene, allyl alcohol, allyl chloride, styrene, cyclohexene and other materials of comparable likeness. The catalysts can also be used in epoxidation where more than one olefin is contained in the gas feed.

For use, the concentration of olefin contained in the reaction gas is considered to be not particularly critical and can be varied over a wide range. In most cases the composition of the gas will depend on the type of reactor used, the relative amount of oxygen and hydrogen used and if required, the amount of diluent added. For commercialization it is envisaged that the total olefin concentration present in the gas stream, entering the reactor will vary but is not limited to, between 5 to 80 mole percent, with the remainder of the gas comprising of oxygen, hydrogen and an optional diluent gas.

The oxygen used in this process may come from any suitable source, such as air. However other sources of oxygen can be used such as nitrogen oxides or ozone. The invention can also function in the presence of hydrogen peroxide. The amount of oxygen required is dependent upon a number of parameters and may vary over a wide range, However, for best results the use of an olefin to oxygen molar ratio of greater than one is considered important. Often the selectivity is seriously reduced in the reactor if oxygen is present in large amounts, with the olefin undergoing either complete or partial oxidation. Typically the amount of oxygen present is usually between 1 and 20 mole percent, although other ratios may and can be used.

The source of hydrogen is also not considered important and may be supplied by any suitable source. By definition any suitable source may include such sources as molecular hydrogen obtained by alkane or alcohol dehydrogenation. The production of molecular hydrogen may be either carried out ex situ or in situ. Or in other words including within the reactor itself. The amount of hydrogen used depends on the amount required to convert the olefin to the corresponding epoxide and is thus variable. Normal operating ranges, however, suggest that the hydrogen concentration contained within the reactor should typically be below 30 mole percent, with the remainder comprising of oxygen olefin and diluent if required.

The addition of diluent is preferred, but is not essential for the reaction of the olefin to take place. The choice of diluent will depend on a number of parameters, including but not limited to safety, cost factors and inertness. Possible gases that could be used as a diluent are nitrogen, helium, argon or any other inert gas. As the process of transport of the reactants to the surface is the most essential parameter, the catalyst may also be employed in the liquid phase. In this case the liquid in which the catalyst is immersed should also be inert and aid as a good medium for transport of the reactant gases to the catalyst surface.

The metals of the lanthanoide series exhibit an atomic number in the range of from 58-71 and include Cerium, Praseodymium, Neodymium, Promethium, Samarium, Europium, Gadolinium, Terbium, Erbium, Dysprosium, Holmium, Erbium, Thulium, Ytterbium, and Lutetium.

For the invention the Lanthanide elements can be introduced in any suitable form. Active catalyst can be obtained using for example (NH₄)₂ Ce (NO₃)₆, Cerium (IV) t-butoxide, Nd(NO₃)₃ Ho(NO₃)₃ · 5H₂O, Europiumchloride hexahydrate, Europium-nitrate pentahydrate, Er(NO₃)₃ · 5 H₂O, Thulium (III) nitrate hexahydrate and the like.

For the purpose of this invention, the actual source of the material is thus diverse and the choice of materials used will ultimately depend on the preparation method used. A further listing of compounds is deemed not to further enhance the understanding of the skilled artisan.

It is also possible to obtain activity form Au supported on Lanthanide metal systems that are diluted in silicates. Such non-limiting examples are ZSM-5; ZSM-11; ZSM-48 and MCM-41, or any materials of similar chemical or physical structures. One may also prepare active catalyst using gas phase routes, or preferably using standard sol-gel preparation routes as described by e.g. L.C. Klein, Ann. Rev. Mar. Sci., 15, p. 227 and following (1985) or those disclosed in DE-A-199 20 753.

As known in the art the above mentioned catalysts can be operated with all standard promoters. For example alkali metals, alkaline earth. For the purpose of this invention the elements in the Lanthanide series, though normally claimed to be promoters are, for this reaction, considered to be catalysts. It is thus specifically claimed that in the presence of gold each element in the Lanthanide series creates a unique and separate catalytic reactor. It is, however, noted that one may reasonably use lanthanoides as a promoter in a catalyst, not containing any of the elements specifically claimed in this patent, if 1) the concentration of lanthanoide used is less than 1% of the active component of the alternative catalyst and 2) the lanthanoides, used as additives, are not more active as measured by turnover frequency, than the active part of the catalyst to which they are added.

Logically, if desired the Lanthanide metal elements can be produced together in any combination, with gold, to create so-called co-catalyst systems. The catalysts may also be included in or bound to other support materials, or catalysts, that act to improve the physical properties of the system. Non limiting example are the use of a secondary support in order to impregnate the catalyst onto a monolith or supports that act to increase the total surface area exposed. Secondary supports may also be used to improve the physical properties such as to control coagulation. Non-limiting examples of such supports include silica, alumina, aluminasilicates, clays, carbonates, zeolites or any combination or mixture of the above.

Though not specific to the current invention it is known in the art that the catalysts can be used in any reactor capable of controlling and mixing the required oxygen, hydrogen and olefin. The reactor can be operated as batch, fixed bed, transport bed, fluidized bed and may be used as prepared, or as a powder, or compressed pellets.

For this invention, the gold and lanthanoide metal loadings are variable. The gold particles in the current invention is observed to normally vary in size from 2 to 400 nm. It is, however, advisable that a high surface area is used for the highest possible conversions. For this reason gold particles of sizes between 1 and 10 nm are usually preferred. As a result typical gold loadings should usually be sufficiently low, i.e. typically below 0.1 atom percent, to facilitate the formation of the smaller nanometer (nm) size clusters. Catalyst comprising of gold with higher than 5 atom percent, though not considered to be optimal, may however be prepared. Techniques for depositing gold at nanometer sizes can be found in WO 98/00413-A1, WO 98/00414-A1, WO 98/00415-A1, WO 97/34692-A1; Haruta et al., J. Catal., 115 pp. 301-309 (1989); Tsubota et al. in "Preparation of Catalyst V" Stud. Surf. Sci. Catal., 63, eds., G. Poncelet et al., Elsevier, PP 695-704 (1991); Kobayashi et al, Sensors and actuators, B1 pp 222-225 (1990); Sakurai and Haruta, Catal. Today, 29 pp .361 (1996); D. Cunningham et al. Res. Chem. Intermediates, 19 pp. 1-13 (1993); Okumura et al., Solid State Ionics, 95 143 (1997); D. Cunningham et al, Catal. Lett., 63 (1-2) pp. 43-47 (1999). As such any process for depositing a metal onto a solid support can be employed, for example impregnation, co-precipitation chemical vapor deposition, ion exchange techniques and deposition-precipitation. For catalyst preparation it is usually recommended that chlorine contamination be limited or avoided. A calcination step is usual, but not always required, and may be carried out either by rapid heat/quenching processing, or alternatively by long term exposure to a heating source. The temperature for calcination required depends on the preparation process but is usually not above 700°C.

One suitable method for obtaining active catalysts is that by sol-gel synthesis. In this process an alkoxide of the required lanthanide metal is added to a suitable silanol compound, such as for example Tetraethylorthosilicate, Hexa-methyldisilazan, Tetra-decyloxysilane, Tetra-butoxysilane, Methyl-tri-ethoxysilane, Tetra-ethoxysilane, Tetra-methoxysilane, or essentially any other suitable silanol, including those containing benzene or more complex organic groups. The silanol is usually diluted in an alcohol such as ethanol, or propanal, butanol, or any suitable alcohol that is a liquid at the temperature of preparation. To this an acidic gold solution is added and the pH adjusted by the use of an acid. The resultant solution is typically homogeneous and forms a gel in which the gold is uniformly dispersed throughout. For the removal of chlorine it has been found adequate to simply heating the gel at elevated temperatures, such as at 350°C. However, for best results it is often best to wash the catalyst repeatedly in water that is free of chlorine or fluorine. The formation of metallic gold particles can occur at any temperature including ambient room temperature. Promoters may be added to the catalysts to increase selectivity or yield, or alternatively to increase the operating life of the catalysts. Known examples include the alkali metals lithium, sodium potassium and rubidium.

During operation it is envisaged that the invention will operate at a temperature from 20°C to 250°C. The actual temperature used will depend upon such factors as; the reaction gas composition, or in the case of liquid reactors the freezing point of the fluid, the yield and degree of selectivity required, the pressure within the reactor, the reactor type used, the type of olefin present and any other parameter which may influence or require the need to modify the operating temperature. Pressure ranges from atmospheric to 200 bar are normally considered suitable. During operation with gaseous mixtures the gas flow rate measured as a space velocity may vary and ultimately will depend upon the reaction parameters used.

Regeneration of the catalysts can be carried out by any one of a number of normal routines, such as high temperature treatment, or washing in a solution of neutral or acidic reagents (DE-A1-198 04 712).

### Examples

### Example 1 Catalyst containing Thulium (Catalyst A1)

One process to obtain catalysts is by sol-gel/deposition precipitation synthesis. This technique is generally adaptable to all elements of the Lanthanoide series. To make a catalyst comprising of Au supported on Thulium/tetraethylorthosilicate 2.92 ml of an alcohol such as ethanol is first mixed with 3298 mg Tetraethylorthosilicate. 0.345 grams of the

Thulium compound, which for the purpose of this example is Thulium(III) nitrate hexahydrate is then added to the mixture. To this mixture 1.67 g HNO₃ dissolved in 600 µl H₂O is added and the sample mixed until gelation occurs. After gelation the sample is then dried, crushed into a powder and heated for 24 hours at 350°C.

To load the gold, 1.0 grams of the Thulium silicate compound produced above is added to 20 ml of water. To the suspension, 0.2 grams of gold chlorauric acid, dissolved in 10 ml water, is added and the suspension mixed for 1 hour. 10 ml of 0.015 molar sodium citrate is then added to the system and the system allowed to mix for a further 1 hour. The wet powder is then removed and repeatedly washed with distilled water to remove chlorine, dried overnight at 100°C, 200mbar and finally calcined at 350°C.

After calcination, 500 mg of catalyst (A1) was then inserted into a gas reactor cell and studied at a temperature of 100°C. For this study a gas comprising of 5.78% propylene 75.65% hydrogen 4.81% oxygen and 13.76% nitrogen dilutant was passed through the bed at a flowrate of space velocity of 3500 ml hr⁻¹/gram.cat. Analysis of the reaction products in the gas phase were analysed by gas chromatography.

**Table 1**

| Catalyst A1 containing 1.0 Atom % Au 5.0 Atom % Tm | Acetaldehyde | Propylene Oxide | Propionaldehyde | Acetone |
|---|---|---|---|---|
| Conversion | 0.000 | 0.009 | 0.000 | 0.003 |
| Selectivity | 0.000 | 74.92 | 0.000 | 25.08 |

Table 1: Distribution of partial oxidation products obtained on passing propylene through a catalyst, comprising of Au, Thulium and Tetraethylorthosilicate (TEOS) prepared by the sol-gel/deposition precipitation technique.

### Example 2-11

The catalysts were prepared in a manner identical to Example 1 with the exception, that the Thulium compounds were replaced by the respective amount of the compounds of Table 2.

**Table 2**

| Example | Tm(III) nitrate replaced by | Tm silicate replaced by |
|---|---|---|
| 2 | Praseodymium(III) chloride | Praseodymium silicate |
| 3 | Samarium(III) nitrate | Samarium silicate |
| 4 | Europium chloride hexahydrate | Europium silicate |
| 5 | Gadolinium(III) 2,2,6,6-tetramethylheptanedionate | Gadolinium silicate |
| 6 | Terbium(III) nitrate | Terbium silicate |
| 7 | Dysprosium(III) nitrate | Dysprosium silicate |
| 8 | Holmium(III)nitrate | Holmium silicate |
| 9 | Erbium methoxyethoxide | Erbium silicate |
| 10 | Ytterbium nitrate pentahydrate | Ytterbium silicate |
| 11 | Lutetium(III) nitrate | Lutetium silicate |

All the materials listed are commercially available.

**Table 3**

| Catalyst of Expl | | Acetaldehyde | Propylene Oxide | Propionaldehyde | Acetone |
|---|---|---|---|---|---|
| 2 5 atom % Pr | % Conversion | 0.000 | 0.014 | 0.000 | 0.023 |
| | % Selectivity | 0.000 | 37.67 | 0.000 | 62.33 |
| 3 5 atom % Sm | % Conversion | 0.000 | 0.014 | 0.000 | 0.025 |
| | % Selectivity | 0.000 | 35.44 | 0.000 | 64.56 |
| 4 5 atom % Eu | % Conversion | 0.007 | 0.012 | 0.023 | 0.028 |
| | % Selectivity | 10.45 | 16.51 | 33.36 | 39.68 |
| 5 5 atom % Gd | % Conversion | 0.000 | 0.009 | 0.003 | 0.001 |
| | % Selectivity | 0.000 | 69,9 | 23.43 | 6.67 |
| 6 5 atom % Tb | % Conversion | 0.000 | 0.007 | 0.002 | 0.004 |
| | % Selectivity | 0.000 | 51.63 | 18.75 | 29.62 |
| 7 5 atom % Dy | % Conversion | 0.007 | 0.014 | 0.026 | 0.021 |
| | % Selectivity | 10.19 | 21.08 | 38.21 | 30.52 |
| 8 5 atom % Ho | % Conversion | 0.000 | 0.011 | 0.005 | 0.012 |
| | % Selectivity | 0.000 | 39.68 | 16.69 | 43.63 |
| 9 5 atom % Er | % Conversion | 0.003 | 0.028 | 0.004 | 0.006 |
| | % Selectivity | 8.12 | 64.89 | 11.06 | 15.93 |
| 10 5 atom % Yb | % Conversion | 0.008 | 0.068 | 0.008 | 0.001 |
| | % Selectivity | 9.15 | 80.36 | 9.11 | 1.38 |
| 11 5 atom % Lu | % Conversion | 0.013 | 0.073 | 0.046 | 0.043 |
| | % Selectivity | 7.64 | 41.59 | 26.29 | 24.48 |

Table 3: Distribution of partial oxidation products obtained on passing propylene through a catalyst prepared by the sol-gel/deposition precipitation technique and comprising of 1.0 atom% Au, Tetraethylorthosilicate (TEOS) and 5 atom% Lanthanoide metal.

## Claims

1. A process for the general epoxidation of olefins, using molecular oxygen and hydrogen, characterized in that, as catalyst, a compound comprising gold, preferably in nanometer size, on a support material, in which the support material contain one or more element(s) from the lanthanoide series having the atomic number from 58 to 71 is applied.

2. A process according to claim 1, characterized in that the support material is free of titanium.

3. A process according to claim 1 and/or 2, characterized in that the olefin is propene.

4. A compound comprising gold, preferably in nanometer size, on a support material, in which the support material contain one or more element(s) selected from the group consisting of the metals having the atomic number 58-71 of which Cerium and Neodymium are excluded.

5. A compound according to claim 4, characterized in that the compound contains no titanium.

6. A process for the preparation of the compound according to claim 4 and/or 5, characterized in that gold particles of nanometer size are deposited on a support material in which the support material contain one or more element(s) from the lanthanoide series.

7. A process according to claim 6, characterized in that the compound contains no titanium.

8. A process for the preparation of the compound according to claim 4 and/or 5, characterized in that the compound is prepared via a sol-gel-process.

9. A process according to claim 8, characterized in that metal nitrates are used for the sol-gel-process.

10. A method of catalyzing a chemical reaction through conducting said chemical reaction in the presence of a compound comprising gold, preferably in nanometer size, on a support material, in which the support material contain one or more element(s) selected from the group consisting of the metals having the atomic number 58-71 of which Cerium and Neodymium are excluded.

11. A method according to claim 10, characterized in that the compound contains no titanium.
